# EUROPÄISCHE PATENTSCHRIFT

(11) **EP 3 838 888 B1**
(45) Veröffentlichungstag und Bekanntmachung des Hinweises auf die Patenterteilung: **30.03.2022**
(21) Anmeldenummer: 19216888.8
(22) Anmeldetag: 17.12.2019
(51) Int. Cl.: C07C 69/608, C08K 5/00, C08K 5/12, C07C 67/02, C08L 27/06, C09D 127/06

(54) **TRIESTER DER CYCLOHEXANTRIPROPIONSÄURE**
TRIESTERS OF CYCLOHEXANTRIPROPIONIC ACID
TRIESTER D'ACIDE CYCLOHEXANTRIPROPIONIQUE

(43) Veröffentlichungstag der Anmeldung: 23.06.2021
(73) Patentinhaber: Evonik Operations GmbH, 45128 Essen (DE)
(72) Erfinder: SCHULZ, Imke, 59348 Lüdinghausen (DE); GRASS, Michael, 45721 Haltern am See (DE); FRANKE, Robert, 45772 Marl (DE); KRAFT, Johannes, 45770 Marl (DE); BELLER, Matthias, 18211 Ostseebad Nienhagen (DE); JACKSTELL, Ralf, 18106 Rostock (DE)
(74) Vertreter: Evonik Patent Association

(56) Entgegenhaltungen:
- US-A1- 2007 123 716
- US-A1- 2018 319 954

## Beschreibung

Die vorliegende Erfindung betrifft Triester der Cyclohexantripropionsäure, deren Herstellung und Verwendung als Weichmacher für Polymere.

Zur Verbesserung der Verarbeitbarkeit sowie zur Anpassung anwendungsrelevanter Eigenschaften an die jeweiligen Anforderungen werden Polymeren Weichmacher zugesetzt. Hierbei stehen zum Erreichen der jeweilig gewünschten Eigenschaften Weichmacher mit unterschiedlichen Wirkungsprofilen zur Verfügung. Zu den wichtigsten Weichmachern für PVC und Vinylchlorid-haltige Copolymere gehören Verbindungen aus der Gruppe der Phthalate.

Die Phthalate haben - unter anderem abhängig von der Kohlenstoffanzahl in den Alkoholteilen der Esterfunktionen - unterschiedliche Eigenschaften und eignen sich dementsprechend mehr oder weniger stark für unterschiedliche Weichmacheranwendungen. Während Phthalate mit kurzkettigen Alkoholteilen, beispielsweise Dibutylphthalate und Dipentylphthalate, eine niedrige und damit vorteilhafte Geliertemperatur aufweisen und deshalb als Schnellgelierer eingesetzt werden, sind diese Phthalate aufgrund ihrer hohen Flüchtigkeit für andere Anwendungen ungeeignet. Phthalate mit längerkettigen Alkoholteilen, beispielsweise Di*^{iso}*nonylphthalat (DINP), weisen zwar schlechtere Geliereigenschaften auf als die leichteren Homologen, profitieren aber gleichzeitig von einer geringeren Flüchtigkeit, welche jedoch für bestimmte Anwendungen, die hohe Temperaturen tolerieren müssen, noch zu hoch ist. Selbst die relativ geringe Flüchtigkeit von Di(tridecyl)phthalat ist nicht niedrig genug, um den Einsatz dieses Phthalates in vielen Hochtemperaturanwendungen zu ermöglichen. Da Phthalate mit mehr als 13 Kohlenstoffatomen im Alkoholteil eine geringe Polymer-Verträglichkeit aufweisen und die entsprechenden Polymer-Phthalat-Gemische zur Entmischung neigen, gibt es keine Vertreter aus der Gruppe der Phthalat-Weichmacher mit mehr als 13 Kohlenstoffatomen im Alkoholteil, welche beispielsweise als Weichmacher in Hochtemperaturkabeln eingesetzt werden könnten.

Aufgrund ihrer im Vergleich zu den Phthalaten geringeren Flüchtigkeit kommen für Hochtemperaturanwendungen Weichmacher aus der Gruppe der Trimellitate zum Einsatz. Das Fachbuch "Plasticizers - Principles and Practice" von A.S. Wilson (The Institute of Materials, 1995, Seiten 166 bis 170) beschreibt Trimellitate mit Alkoholteilen enthaltend 7 bis 9 Kohlenstoffatome oder Gemische aus C6- und C8-Estern oder C7-, C8- und C9-Estern der Trimellitsäure als kommerziell interessant, wobei Tri(2-ethylhexyl)trimellitat als das wichtigste Trimellitat herausgestellt wird. Selbst diese Trimellitate sind für den Einsatz in Hochtemperaturkabeln oftmals noch zu flüchtig.

Die Offenlegungsschrift US 2018/0319954 A1 offenbart Trimellitate und neben diesen auch Cyclohexan-1,2,4-tricarboxylate und deren Eignung als Weichmacher.

Der vorliegenden Erfindung lag nun die Aufgabe zugrunde, eine neue Gruppe an Weichmachern bereitzustellen, deren Vertreter einen breiten Bereich an Eigenschaften überstreichen, also für viele unterschiedliche Anwendungen einsetzbar sind. Diese neue Weichmachergruppe sollte vorzugsweise sowohl Vertreter mit sehr guter Gelierfähigkeit als auch Vertreter mit exzellenten Hochtemperatureigenschaften umfassen. Bevorzugterweise sollten Vertreter der neuen Weichmachergruppe den Trimellitaten in Hochtemperaturanwendungen wie Kabeln überlegen sein.

Diese Aufgabe wird gelöst durch Triester der Cyclohexantripropionsäure gemäß Anspruch 1. Gegenstand der vorliegenden Erfindung sind Triester der Cyclohexantripropionsäure, in welchen die drei Alkoholteile der drei Estergruppen jeweils 2, 3, 4, 5, 6, 7, 8, 9, 10, 11 oder 12 Kohlenstoff-Atome umfassen.

Triester der Cyclohexantripropionsäure werden wie alle Carbonsäureester formal aus Carbonsäure und Alkohol gebildet, wodurch der Ester einen Säureteil und einen Alkoholteil umfasst.

Erfindungsgemäße Triester der Cyclohexantripropionsäure setzen sich aus dem Cyclohexantripropionsäure-Teil und drei Alkoholteilen zusammen. Diese Alkoholteile enthalten jeweils 2, 3, 4, 5, 6, 7, 8, 9, 10, 11 oder 12 Kohlenstoff-Atome.

Erfindungsgemäße Triester der Cyclohexantripropionsäure werden im Folgenden auch abgekürzt als erfindungsgemäße Triester bezeichnet.

Überraschend wurde gefunden, dass Vertreter dieser neuen Weichmachergruppe für Plastisolanwendungen geeignet sind, eine niedrige Geliertemperatur aufweisen und vorteilhaft als Schnellgelierer eingesetzt werden können, während andere Vertreter dieser Gruppe vorteilhaft in thermoplastischen Anwendungen Verwendung finden können. Vertreter dieser Weichmachergruppe weisen in Plastisol- und in thermoplastischen Anwendungen einen geringen Massenverlust auf. Auch eine gute Tieftemperaturflexibilität (niedrige Glasübergangstemperatur) kann mit Vertretern dieser Gruppe erreicht werden.

Neben der angestrebten Eignung von Vertretern der neuen Weichmachergruppe für Hochtemperaturanwendungen finden sich in der Gruppe Vertreter, welche in Plastisolanwendungen eine geringe Viskosität samt vorteilhaftem Eindickverhalten des betrachteten Plastisols ermöglichen.

Die drei Propionsäureester-Reste erfindungsgemäßer Ester können an verschiedenen Positionen des Cyclohexanringes angebunden sein. Bevorzugt handelt es sich bei den erfindungsgemäßen Triestern jedoch um Triester der Cyclohexan-1,2,4-tripropionsäure oder um Triester der Cyclohexan-1,3,5-tripropionsäure, insbesondere um Triester der Cyclohexan-1,2,4-tripropionsäure.

Die Alkoholteile der Triester der Cyclohexantripropionsäure können cyclische oder acyclische Alkylreste mit oder ohne funktionelle Gruppen einschließlich Mehrfachbindungen sein. Hierbei ist es unerheblich, ob die funktionellen Gruppen aus dem zur Herstellung der erfindungsgemäßen Triester eingesetzten Alkohol stammen oder nachträglich in das Triester-Molekül eingefügt wurden. Möglich sind auch Alkoholteile, welche aromatische Ringe umfassen, die wiederum keine, eine oder mehrere funktionellen Gruppen tragen. Bevorzugt enthalten die Alkoholteile erfindungsgemäßer Triester neben dem Sauerstoff der Esterfunktion keine weiteren Heteroatome und keine Mehrfachbindungen. Diese formal auf Alkanolen, vorzugsweise auf acyclischen Alkanolen basierenden Alkoholteile haben den Vorteil, dass die Herstellung der resultierenden Triester aufgrund der Verfügbarkeit der Alkanole besonders kostengünstig möglich ist.

Ein bevorzugter Gegenstand der vorliegenden Erfindung sind Triester der Cyclohexantripropionsäure, in welchen die drei Alkoholteile der drei Estergruppen jeweils 2 bis 9, bevorzugt 4 bis 9 oder 4, 5, 6, 7, 8 oder 9 Kohlenstoff-Atome umfassen. Diese Triester weisen gute Gelierungseigenschaften auf und die unter ihrem Einsatz hergestellten Plastisole zeichnen sich durch eine geringe Plastisolviskosität aus, welche zudem über die Zeit nur geringfügig ansteigt. In Plastisolanwendungen, beispielsweise aus Folien, tritt an der Luft nur ein geringer Massenverlust auf. Wie aus den niedrigen Glasübergangstemperaturen ersichtlich, ist die Tieftemperaturflexibilität von Prüfkörpern, welche diese Triester enthalten, höher als bei Vergleichsverbindungen. Bevorzugt handelt es sich bei den erfindungsgemäßen Triestern jedoch um Triester der Cyclohexan-1,2,4-tripropionsäure oder um Triester der Cyclohexan-1,3,5-tripropionsäure, insbesondere um Triester der Cyclohexan-1 ,2,4-tripropionsäure, deren Alkoholteil formal vorzugsweise aus einem acyclischen Alkanol stammt.

Ein anderer bevorzugter Gegenstand der vorliegenden Erfindung sind Triester der Cyclohexantripropionsäure, in welchen die drei Alkoholteile der drei Estergruppen jeweils 7 bis 12, bevorzugt 8 bis 10 und insbesondere 8 oder 9 Kohlenstoff-Atome umfassen. Diese Triester zeichnen sich durch einen sehr geringen Massenverlust bei erhöhten Temperaturen aus und sind deshalb sehr gut für Hochtemperaturanwendungen geeignet. Zusätzlich ist die Tieftemperaturflexibilität von Prüfkörpern, welche diese Triester enthalten, höher als bei Vergleichsverbindungen. Bevorzugt handelt es sich bei den erfindungsgemäßen Triestern jedoch um Triester der Cyclohexan-1,2,4-tripropionsäure oder um Triester der Cyclohexan-1,3,5-tripropionsäure, insbesondere um Triester der Cyclohexan-1,2,4-tripropionsäure, deren Alkoholteil formal vorzugsweise aus einem acyclischen Alkanol stammt.

Bevorzugt enthalten erfindungsgemäße Triester innerhalb eines Moleküls zwei oder drei Alkoholteile mit identischer Summenformel. Hierbei können die "Summenformel-identischen" Alkoholteile innerhalb eines Triesters dieselbe Anordnung der enthaltenen Atome aufweisen, oder sich in der Struktur unterscheiden, also isomere Alkoholteile sein. Vorzugsweise weisen sämtliche in einem Molekül der erfindungsgemäßen Triester enthaltenen Alkoholteile identische Summenformeln und gleichzeitig identische oder unterschiedliche Konstitutionsformeln auf. Erfindungsgemäße Triester, deren Alkoholteile identische Summenformeln und dabei unterschiedliche Konstitutionsformeln aufweisen, enthalten isomere Alkoholreste. Solche Triester sind vorteilhafterweise auch bei tiefen Temperaturen flüssig.

Bevorzugte Triester der Cyclohexantripropionsäure, d. h. Triester der Cyclohexan-1,2,4-tripropionsäure, weisen die in Formel I wiedergegebene Struktur auf, wobei die Reste R identische Summenformeln und gleichzeitig identische oder unterschiedliche Konstitutionsformeln aufweisen.

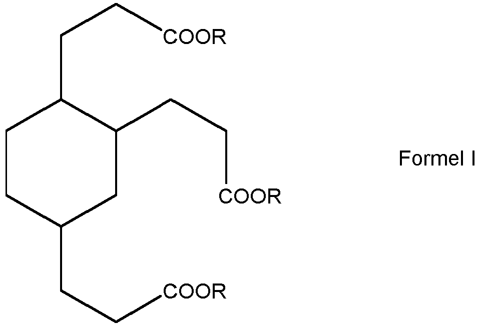

In einer Ausführungsform sind die Reste R der Formel I acylische Alkylreste mit 2 bis 8 oder 9, insbesondere mit 4, 5, 6 oder 7 Kohlenstoffatomen. In einer anderen Ausführungsform sind die Reste R der Formel I acyclische Alkylreste mit 7 bis 10, insbesondere mit 8 oder 9 Kohlenstoffatomen.

Ein weiterer Gegenstand der vorliegenden Erfindung sind Mischungen von mindestens zwei erfindungsgemäßen Triestern der Cyclohexantripropionsäure. Hierbei können sich die mindestens zwei erfindungsgemäßen Triester in ihren Summenformeln, in ihren Konstitutionsformeln oder in beidem unterscheiden. Ein Beispiel für eine Mischung, in welcher der Unterschied in der Summenformel besteht und die Mischung mindestens zwei erfindungsgemäße Triester enthält, ist eine Mischung umfassend Tri(*ⁿ*pentyl)-cyclohexan-1,2,4-tripropionat und Tri(2-ethylhexyl)-cyclohexan-1,2,4-tripropionat. Unterscheiden sich die mindestens 2 erfindungsgemäßen Ester durch ihre Konstitutionsformel, kann beispielsweise in der Mischung mindestens ein Triester der Cyclohexan-1,2,4-tripropionsäure und mindestens ein Triester der Cyclohexan-1,3,5-tripropionsäure enthalten sein. Alternativ oder auch zusätzlich zu etwaigen Unterschieden in der Position der Propionsäureester-Reste am Cyclohexanring kann die Mischung mindestens zwei erfindungsgemäße Triester umfassen, welche "Summenformel-identische" Alkoholteile unterschiedlicher Konstitution, also isomere Alkoholteile, enthalten. Beispielsweise kann eine Mischung einen erfindungsgemäßen Triester enthalten, dessen Alkoholteile ausnahmslos linear sind und einen erfindungsgemäßen Triester umfassen, dessen Alkoholteile einheitlich verzweigt sind. Beispiel einer solchen Mischung ist die Kombination von Tri(*ⁿ*pentyl)-cyclohexan-1,2,4-tripropionat und Tri(2-methylbutyl)-cyclohexan-1,2,4-tripropionat. Möglich sind auch komplexere Mischungen welche Tri(*ⁿ*butyl)-cyclohexan-1,2,4-tripropionat, Tri(*^{iso}*pentyl)-cyclohexan-1,2,4-tripropionat, Tri(*ⁿ*butyl)-cyclohexan-1,3,5-tripropionat und Tri(*^{iso}*pentyl)-cyclohexan-1,3,5-tripropionat umfassen.

Die Vorsilbe *"iso"* kennzeichnet, dass es sich um ein Isomerengemisch mit gemeinsamer Kohlenstoffanzahl handelt. Ein *^{iso}*Pentyl-Rest enthält also mindestens zwei isomere Alkylreste mit 5 Kohlenstoffatomen, wobei in dieser Bezeichnung keine Information dazu enthalten ist, wie viele und welche isomeren Reste in welchem Mengenverhältnis enthalten sind. Sobald in einem Isomerengemisch Tri(*^{iso}*alkyl)cyclohexantripropionat nicht ausschließlich identische erfindungsgemäße Triester enthalten sind, handelt es sich demnach um eine Mischung von mindestens zwei erfindungsgemäßen Triestern, welche sich in ihren Konstitutionsformeln unterscheiden.

Bevorzugte erfindungsgemäße Triester oder erfindungsgemäße Mischungen sind Tri(*ⁿ*butyl)-cyclohexan-1,2,4-tripropionat, Tri(methylpropyl)-cyclohexan-1,2,4-tripropionat, Tri(*ⁿ*pentyl)-cyclohexan-1,2,4-tripropionat, Tri(*^{iso}*pentyl)-cyclohexan-1,2,4-tripropionat, Tri(2-methylbutyl)-cyclohexan-1,2,4-tripropionat, Tri(3-methylbutyl)-cyclohexan-1,2,4-tripropionat, Tri(*ⁿ*hexyl)-cyclohexan-1,2,4-tripropionat, Tri(*^{iso}*hexyl)-cyclohexan-1,2,4-tripropionat, Tri(*ⁿ*heptyl)-cyclohexan-1,2,4-tripropionat, Tri(*^{iso}*heptyl)-cyclohexan-1,2,4-tripropionat, Tri(*ⁿ*octyl)-cyclohexan-1,2,4-tripropionat, Tri(*^{iso}*octyl)-cyclohexan-1,2,4-tripropionat, Tri(2-ethylhexyl)-cyclohexan-1,2,4-tripropionat, Tri(*ⁿ*nonyl)-cyclohexan-1,2,4-tripropionat, Tri(*^{iso}*nonyl)-cyclohexan-1,2,4-tripropionat, Tri(*ⁿ*decyl)-cyclohexan-1,2,4-tripropionat, Tri(*^{iso}*decyl)-cyclohexan-1,2,4-tripropionat, Tri(2-propylheptyl)-cyclohexan-1,2,4-tripropionat, Tri(*ⁿ*butyl)-cyclohexan-1,3,5-tripropionat, Tri(methylpropyl)-cyclohexan-1,3,5-tripropionat, Tri(*ⁿ*pentyl)-cyclohexan-1,3,5-tripropionat, Tri(*^{iso}*pentyl)-cyclohexan-1,3,5-tripropionat, Tri(2-methylbutyl)-cyclohexan-1,3,5-tripropionat, Tri(3-methylbutyl)-cyclohexan-1,3,5-tripropionat, Tri(*ⁿ*hexyl)-cyclohexan-1,3,5-tripropionat, Tri(*^{iso}*hexyl)-cyclohexan-1,3,5-tripropionat, Tri(*ⁿ*heptyl)-cyclohexan-1,3,5-tripropionat, Tri(*^{iso}*heptyl)-cyclohexan-1,3,5-tripropionat, Tri(*ⁿ*octyl)-cyclohexan-1,3,5-tripropionat, Tri(*^{iso}*octyl)-cyclohexan-1,3,5-tripropionat, Tri(2-ethylhexyl)-cyclohexan-1,3,5-tripropionat, Tri(*ⁿ*nonyl)-cyclohexan-1,3,5-tripropionat, Tri(*^{iso}*nonyl)-cyclohexan-1,3,5-tripropionat, Tri(*ⁿ*decyl)-cyclohexan-1,3,5-tripropionat, Tri(*^{iso}*decyl)-cyclohexan-1,3,5-tripropionat und Tri(2-propylheptyl)-cyclohexan-1,3,5-tripropionat.

Wie bereits beschrieben, weisen erfindungsgemäße Ester vorteilhafte Eigenschaften beim Einsatz als Weichmacher für Polymere auf. Ein weiterer Gegenstand der vorliegenden Erfindung ist deshalb ein Weichmacher für Polymere umfassend einen erfindungsgemäßen Triester oder eine erfindungsgemäße Mischung (umfassend mindestens zwei dieser Triester) und optional mindestens eine weitere Polymer-weichmachende Verbindung. Besonders gut ist dieser Weichmacher für PVC geeignet.

Gegenstand der vorliegenden Erfindung ist zudem eine Zusammensetzung, enthaltend einen erfindungsgemäßen Triester oder eine erfindungsgemäße Mischung (umfassend mindestens zwei dieser Triester) oder einen erfindungsgemäßen Weichmacher und ein oder mehrere Polymere.

Geeignete Polymere sind vorzugsweise ausgewählt aus der Gruppe, die gebildet wird durch Polyvinylchlorid (PVC), Homo- oder Copolymere auf Basis von Ethylen, Propylen, Butadien, Vinylacetat, Glycidylacrylat, Glycidylmethacrylat, Ethylacrylat, Butylacrylat oder Methacrylat mit Alkoxyresten von verzweigten oder unverzweigten Alkoholen mit einem bis zehn Kohlenstoffatom(en), Acrylnitril oder cyclischen Olefinen, Polyvinylidenchlorid (PVDC), Polyacrylaten, insbesondere Polymethylmethacrylat (PMMA), Polyalkylmethacrylat (PAMA), Polyharnstoffe, silylierte Polymere, Fluorpolymere, insbesondere Polyvinylidenfluorid (PVDF), Polytetrafluorethylen (PTFE), Polyvinylacetat (PVAc), Polyvinylalkohol (PVA), Polyvinylacetale, insbesondere Polyvinylbutyral (PVB), Polystyrolpolymere, insbesondere Polystyrol (PS), expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und Cellulose-Derivate, insbesondere Nitrocellulose (NC), Ethylcellulose (EC), Celluloseacetat (CA), Cellulose-Acetat/Butyrat (CAB), Gummi und Silikone.

In einer bevorzugten Ausführungsform ist mindestens ein Polymer bzw. sind vorzugsweise mindestens 90 Gew.-% der Polymere in der Zusammensetzung ausgewählt aus der Gruppe bestehend aus Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat.

Die Menge an erfindungsgemäßem Triester in der erfindungsgemäßen ein oder mehrere Polymere enthaltenden Zusammensetzung beträgt vorzugsweise 5 bis 150 Massenteile, bevorzugt 10 bis 120 Massenteile, besonders bevorzugt 15 bis 110 Massenteile und ganz besonders bevorzugt 20 bis 100 Massenteile pro 100 Massenteile Polymer. Es sind aber auch ein oder mehrere Polymere enthaltende Zusammensetzungen denkbar, die weniger als 20 Massenteile erfindungsgemäßen Triester pro 100 Massenteile Polymer umfassen.

Die erfindungsgemäße Zusammensetzung ist mit Vorzug Bestandteil eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusions- oder Spritzgussartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

Ein weiterer Gegenstand der vorliegenden Erfindung ist die Verwendung eines erfindungsgemäßen Triesters oder einer erfindungsgemäßen Mischung (umfassend mindestens zwei dieser Triester) als Weichmacher für Polymere. Bevorzugt wird der erfindungsgemäße Triester, die erfindungsgemäße Mischung (umfassend mindestens zwei dieser Triester) als Weichmacher für die oben bereits genannten Polymere, insbesondere für Polyvinylchlorid (PVC), Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat eingesetzt. Besonders bevorzugt ist die Verwendung als Weichmacher für Polyvinylchlorid (PVC).

Diese Verwendung resultiert mit Vorzug im Einsatz eines erfindungsgemäßen Triesters oder einer erfindungsgemäßen Mischung (umfassend mindestens zwei dieser Triester) in Klebstoffen, Dichtungsmassen, Beschichtungsmassen, Lacken, Farben, Plastisolen, Schäumen, Kunstleder, Fußbodenbelägen, insbesondere Deck- und Schaumschicht, Dachbahnen, Unterbodenschutz, Gewebebeschichtungen, Kabeln, Drahtisolierungen, Schläuchen, Extrusionsartikeln, Folien, im Automobilinnenbereich, in Tapeten, Tinten, Spielzeug, Kontaktfolien, Lebensmittelverpackungen oder medizinischen Artikeln, insbesondere in Schläuchen oder Blutbeuteln.

In einer bevorzugten Ausführungsform werden Triester der Cyclohexantripropionsäure, in welchen die drei Alkoholteile der drei Estergruppen 7 bis 12, bevorzugt 8 bis 10 und insbesondere 8 oder 9 Kohlenstoff-Atome umfassen, als Polymer-weichmachende Verbindung in Hochtemperaturanwendungen, insbesondere Hochtemperaturkabeln oder Armaturenbrettbestandteilen verwendet.

In einer anderen bevorzugten Ausführungsform finden Triester der Cyclohexantripropionsäure, in welchen die drei Alkoholteile der drei Estergruppen 2 bis 9, bevorzugt 4 bis 9 oder 4, 5, 6, 7, 8 oder 9 Kohlenstoff-Atome umfassen, als Polymer-weichmachende Verbindung in Plastisol-Anwendungen Verwendung. Bevorzugt ist die Anwendung in Gewebebeschichtungen, Tapeten, Kunstleder sowie in Folien, Dachbahnen und Bodenbelägen.

Die erfindungsgemäßen Triester können hergestellt werden durch
- Kernhydrierung der korrespondierenden Triester der Benzoltripropionsäure,
- Umesterung vom Trialkylester der Cyclohexantripropionsäure mit mindestens einem Alkohol umfassend 2, 3, 4, 5, 6, 7, 8, 9, 10, 11 oder 12 Kohlenstoff-Atome, wobei der Alkohol des einzubauenden Alkoholteils des Trialkylesters höher siedet als der Alkohol des Alkoholteils, der im Rahmen der Umesterung ersetzt werden soll,
- Veresterung von Cyclohexantripropionsäure mit mindestens einem Alkohol umfassend 2, 3, 4, 5, 6, 7, 8, 9, 10, 11 oder 12 Kohlenstoff-Atome oder
- Alkoxycarbonylierung von Trivinylcyclohexan mit mindestens einem Alkohol umfassend 2, 3, 4, 5, 6, 7, 8, 9, 10, 11 oder 12 Kohlenstoff-Atome.

Mit Vorzug wird bei der Umesterung der Trimethylester oder der Triethylester eingesetzt.

Bevorzugter Gegenstand der vorliegenden Anmeldung ist die Herstellung erfindungsgemäßer Triester der Cyclohexantripropionsäure
- durch Kernhydrierung von Triestern der Benzoltripropionsäure, in denen die Alkoholteile der Estergruppen jeweils 2, 3, 4, 5, 6, 7, 8, 9, 10, 11 oder 12 Kohlenstoff-Atome umfassen,
- Umesterung vom Trialkylester der Cyclohexantripropionsäure mit mindestens einem Alkohol umfassend 2, 3, 4, 5, 6, 7, 8, 9, 10, 11 oder 12 Kohlenstoff-Atome, wobei der Alkohol des einzubauenden Alkoholteils des Trialkylesters höher siedet als der Alkohol des Alkoholteils, der im Rahmen der Umesterung ersetzt werden soll,
- durch Veresterung von Cyclohexantripropionsäure mit mindestens einem Alkohol umfassend 2, 3, 4, 5, 6, 7, 8, 9, 10, 11 oder 12 Kohlenstoff-Atome oder
- durch Alkoxycarbonylierung von Trivinylcyclohexan mit mindestens einem Alkohol umfassend 2, 3, 4, 5, 6, 7, 8, 9, 10, 11 oder 12 Kohlenstoff-Atome.

Besonders bevorzugt ist die Herstellung der Triester der Cyclohexan-1,2,4-tripropionsäure unter Einsatz der jeweiligen Cyclohexan-1,2,4-Verbindungen.

Mit Vorzug wird bei der Umesterung der Trimethylester oder der Triethylester eingesetzt.

Ein besonders bevorzugter Gegenstand der vorliegenden Erfindung ist die Herstellung erfindungsgemäßer Triester der Cyclohexan-1,2,4-tripropionsäure
- durch Kernhydrierung von Triestern der Benzol-1,2,4-tripropionsäure, in denen die Alkoholteile der Estergruppen jeweils 2, 3, 4, 5, 6, 7, 8, 9, 10, 11 oder 12 Kohlenstoff-Atome umfassen,
- durch Umesterung des Trimethylesters oder des Triethylesters der Cyclohexan-1,2,4-tripropionsäure mit mindestens einem Alkohol umfassend 2, 3, 4, 5, 6, 7, 8, 9, 10, 11 oder 12 Kohlenstoff-Atome,
- durch Veresterung von Cyclohexan-1,2,4-tripropionsäure mit mindestens einem Alkohol umfassend 2, 3, 4, 5, 6, 7, 8, 9, 10, 11 oder 12 Kohlenstoff-Atome oder
- durch Alkoxycarbonylierung von 1,2,4-Trivinylcyclohexan mit mindestens einem Alkohol umfassend 2, 3, 4, 5, 6, 7, 8, 9, 10, 11 oder 12 Kohlenstoff-Atome.

Die Kernhydrierung der Triester der Benzol-1,2,4-tripropionsäure zu den erfindungsgemäßen Triestern kann in einem oder mehreren hintereinandergeschalteten Hydriereinheiten durchgeführt werden. Die Hydriereinheiten bestehen vorzugsweise jeweils aus mindestens einem, vorzugsweise mehreren Hydrierreaktor(en). Dieser mindestens eine Hydrierreaktor kann ein Rohrreaktor, Rohrbündelreaktor oder vorzugsweise ein Schachtofen sein. Die einzelnen Reaktoren können adiabatisch, polytrop oder praktisch isotherm, d.h. mit einem Temperaturanstieg von typischerweise kleiner als 10 °C, betrieben werden. Dabei werden insbesondere die in Schlaufenfahrweise betriebenen Reaktoren bevorzugt quasi isotherm gefahren, vorzugsweise mit einem Temperaturanstieg kleiner 10 °C, besonders bevorzugt kleiner 5 °C betrieben. Eine oder mehrere der Hydriereinheiten können in Schlaufenfahrweise betrieben werden.

Vorzugsweise wird die Hydrierung der Triester der Benzoltripropionsäure kontinuierlich und an festen, im Festbett angeordneten Katalysatoren mit einem Wasserstoff-haltigen Gas durchgeführt.

Als Hydriergase können beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Die Verwendung von Inertgasen ist optional, bevorzugt wird Wasserstoff in einer Reinheit größer 95 %, insbesondere größer 98 % eingesetzt. Inertgasanteile können beispielsweise Stickstoff oder Methan sein.

Mit Vorzug werden feste Hydrierkatalysatoren eingesetzt, die mindestens ein Metall der achten Nebengruppe des Periodensystems der Elemente enthalten. Bevorzugt werden als Aktivmetalle der achten Nebengruppe des Periodensystems der Elemente Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird. Neben den bereits genannten Metallen kann zusätzlich mindestens ein Metall der ersten und/oder siebten Nebengruppe des Periodensystems der Elemente in den Katalysatoren enthalten sein. Bevorzugt wird Rhenium und/oder Kupfer eingesetzt. Die eingesetzten Katalysatoren sind bevorzugt Trägerkatalysatoren. Als Träger können beispielsweise folgende Stoffe verwendet werden: Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische. Besonders bevorzugt wird ein Katalysator eingesetzt, der einen Aluminiumoxid oder einen Titandioxid-Träger aufweist. Zusätzlich können diese Trägermaterialien Alkalimetalle, Erdalkalimetalle und/oder Schwefel enthalten. Es werden bevorzugt Ruthenium-Katalysatoren eingesetzt.

Das Hydrierverfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und/oder einer hohen Raum-Zeit-Ausbeute werden die in Schlaufenfahrweise betriebenen Reaktoren vorzugsweise mit hohen Flüssigkeitsbelastungen von 10 bis 400, bevorzugt von 20 bis 200 und besonders bevorzugt von 40 bis 150 m³ pro m² Querschnitt des leeren Reaktors und Stunde gefahren.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten. Ganz besonders bevorzugt wird das Produkt der Hydrierung als Lösemittel verwendet.

Sowohl die Umesterung vom Trimethylester der Cyclohexantripropionsäure mit mindestens einem Alkohol umfassend 2, 3, 4, 5, 6, 7, 8, 9, 10, 11 oder 12 Kohlenstoff-Atome als auch die Veresterung der Cyclohexantripropionsäure mit einem oder mehreren solcher Alkohole wird vorzugsweise in Anwesenheit eines Katalysators oder mehrerer Katalysatoren, beispielsweise unter Verwendung von Brönstedt- oder Lewis-Säuren oder -Basen als Katalysator, durchgeführt. Als besonders geeignete Katalysatoren haben sich Schwefelsäure, Methansulfonsäure, p-Toluolsulfonsäure, Metalle oder deren Verbindungen erwiesen. Beispiele für besonders bevorzugte Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat sowie Natriummethanolat und Kaliummethanolat. Die Cyclohexantripropionsäure ist über Hydroxycarbonylierung, also die Edelmetall-katalysierte Umsetzung von Trivinylcyclohexan mit CO und H₂O, zugänglich.

Das Ver- und das Umesterungsverfahren kann in dem Fachmann bekannten, typischen Veresterungsapparaturen unter üblichen Verfahrensbedingungen durchgeführt werden. Vorzugsweise findet das Verfahren bei Temperaturen bei oder oberhalb des Siedepunktes des bei der Reaktion gebildeten Alkohols statt, so dass dieser aus dem Reaktionsgemisch abdestilliert werden kann. Beispiele geeigneter Umesterungsverfahren sind im Beispielteil beschrieben.

Das Veresterungsverfahren beziehungsweise das Umesterungsverfahren wird vorzugsweise bei einer Temperatur von 100 bis 300°C, bevorzugt bei 120 bis 270°C und insbesondere bei 140 bis 250°C durchgeführt. Der Druck innerhalb der Veresterungsapparatur liegt vorzugsweise bei 0,1 bis 20 oder 15 bar, insbesondere bei 0,1 bis 10 bar.

Bevorzugt ist ein Alkoxycarbonylierungsverfahren umfassend die Verfahrensschritte:
a) Vorlegen einer der Verbindungen (i), (ii), (iii) oder einem Gemisch aus mindestens zwei dieser Verbindungen; 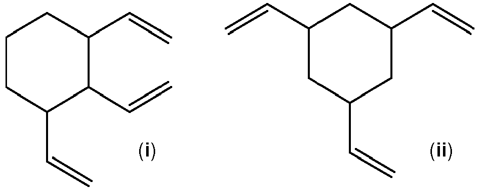 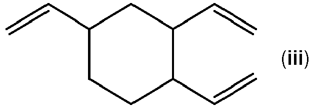
b) Zugabe des Liganden (L) und einer Verbindung, welche Pd umfasst, oder ein Komplex umfassend Pd und den Liganden (L); 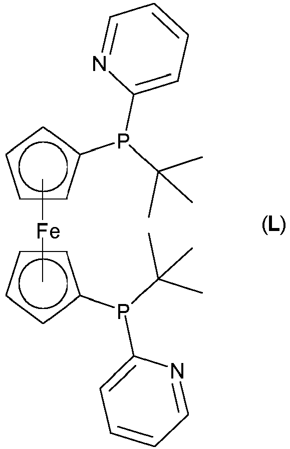
c) Zugabe eines Alkohols mit 1 bis 12 Kohlenstoff-Atomen;
d) Zuführen von CO;
e) Erwärmen des Reaktionsgemisches aus a) bis d), wobei die Verbindung / das Gemisch aus a) zu einem Triester umgesetzt wird.

In einer Variante des Verfahrens wird im Verfahrensschritt a) die Verbindung (i) vorgelegt, in einer anderen Variante die Verbindung (ii). Bevorzugt enthält der Alkohol in Verfahrensschritt c) neben dem Sauerstoff keine weiteren Heteroatome und keine Mehrfachbindungen und ist insbesondere ausgewählt aus Methanol, Ethanol, "Butanol, Methylpropanol, "Pentanol, *^{iso}*Pentanol, 2-Methylbutanol, 3-Methylbutanol, "Hexanol, *^{iso}*Hexanol, "Heptanol, *^{iso}*Heptanol, *ⁿ*Octanol, *^{iso}*Octanol, 2-Ethylhexanol, "Nonanol, *^{iso}*Nonanol, "Decanol, *^{iso}*Decanol und 2-Propylheptanol.

Besonders bevorzugt wird durch Methoxycarbonylierung der Trimethylester hergestellt und dieser dann zum erfindungsgemäßen Triester oder zum Gemisch erfindungsgemäßer Triester umgeestert.

In der Alkoxycaronylierung erfolgt das Zuführen von CO in Verfahrensschritt d) vorzugsweise bis zu einem Druck im Bereich von 20 bar bis 60 bar und insbesondere bei 30 bar bis 50 bar. Die Temperatur im Verfahrensschritt e) liegt mit Vorzug im Bereich von 90 °C bis 130 °C, insbesondere im Bereich von 100 °C bis 120 °C.

Anschließend an die Alkoxycarbonylierung wird der Triester bevorzugterweise in einem Schritt f) aufgereinigt.

Experimenteller Teil:

### Säurezahl:

Die Säurezahl wurde in Anlehnung an DIN EN ISO 2114 bestimmt.

### GC-Analysen:

Die GC- Analyse erfolgte mit folgenden Parametern:
Kapillarsäule: 30 m DB5; 0,25 mm ID; 0,25 µm Film
Trägergas: Helium
Säulenvordruck: 80 kPa
Split: ca. 23,8 ml/min
Ofentemperaturprogramm (Dauer: 51 min): 50 °C (für 1 min), Aufheizen mit 7,5 °C/min auf 350 °C (Temperatur halten für 30 min)
Injektor: 350 °C
Detektor (FID): 400 °C
Injektionsvolumen: 1,0 µl

Die Identifizierung der Komponenten im Chromatogramm der Probe erfolgte mittels einer Vergleichslösung der relevanten Ester. Im Anschluss erfolgte eine Normierung der Signale im Chromatogramm der Probe auf 100 Flächen-%. Die Stoffmengenverhältnisse wurden in hinreichender Näherung aus den Flächenverhältnissen der einzelnen Signale bestimmt.

Die Reinheit wurde über den Anteil der Produktsignale an den Gesamtflächen im Chromatogramm bestimmt.

### Beispiel 0: Herstellung Trimethyl-cyclohexan-1,2,4-tripropionat

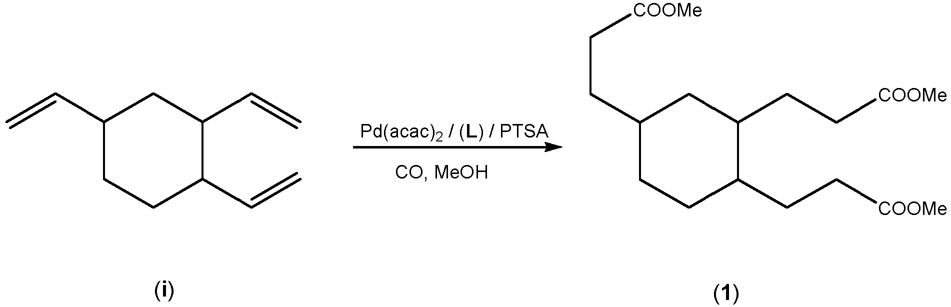

[Pd(acac)₂] (15,2 mg, 0,1 mol%), (L) (Formel siehe oben, 103 mg, 0,4 mol%) und Paratoluolsulfonsäure (PTSA, 143 mg, 1,5 mol%) wurden unter einer Argonatmosphäre in einen 100-ml-Stahlautoklaven gegeben. Dann wurden Methanol (MeOH, 30 ml) und Trivinylcyclohexan (i) (8,1 g, 50 mmol) mittels einer Spritze injiziert. Der Autoklav wurde dreimal mit CO gespült und anschließend mit einem CO-Druck von 40 bar beaufschlagt. Die Reaktion erfolgte über 10 h bei 110 °C. Danach wurde der Autoklav auf Raumtemperatur abgekühlt und entspannt. Das gewünschte Produkt wurde durch Destillation (165 °C bei 10⁻³ bar) aufgereinigt und mit ¹H-, ¹³C-NMR und HR-MS charakterisiert (15,6 g, Ausbeute 91%, Reinheit 98%).

¹H-NMR (300 MHz, C₆D₆) δ = 3,39-3,37 (m, 9H), 2,24-1,86 (m, 7H), 1,48-0,27 (m, 14 H). ¹³C-NMR (75 MHz, C₆D₆) δ = 173,68-173,54 (m), 51,04, 40,60-40,47 (m), 38,24, 38,14, 37,51, 37,07, 36,54, 36,10, 35,52, 35,14, 33,87, 32,70, 32,55, 32,51, 32,38, 32,29, 32,23, 32,08, 31,97, 31,86, 31,76, 31,68, 31,63, 31,43, 30,98, 30,79, 30,75, 29,31, 28,52, 28,47, 28,34, 28,13, 28,11, 27,13, 26,58, 25,12, 20,79, 19,74.

MS (EI): 311 (13,40), 293 (3,65), 269 (75,76), 237 (60,40), 219 (25,13), 205 (100), 191 (17,62), 177 (14,83), 145 (24,59).

HR-MS (ESI): Berechnet C₁₈H₃₀O₆ [M + H]⁺: 343,21152, Gefunden: 343,21113.

### Beispiele 1 - 5: Herstellung erfindungsgemäßer Trialkylcyclohexan-1,2,4-tripropionate

In eine Destillations-Apparatur umfassend Tauchrohr, Thermometer und eine Raschigringkolonne mit aufgesetztem Kondensator wurde die Menge mₑ Trimethyl-cyclohexan-1,2,4-tripropionat vorgelegt und in der Menge mₐ des Alkohols suspendiert. Die Apparatur wurde mindestens eine Stunde mit Stickstoff (6 I/h) über das Tauchrohr gespült, bevor 0,15 Massen-% Tetra-"butyltitanat (Sigma Aldrich, Reinheit > 97 %), bezogen auf die Masse des Tripropionats, hinzugegeben wurden. Während einer bis zur Beendigung der Reaktion andauernden Einperlung von Stickstoff (6 I/h) wurde das Gemisch langsam unter Rühren zum Sieden erhitzt. Ab 61 bis 63 °C Kopftemperatur fiel Methanol an, welches kontinuierlich über den Destillationskopf aus der Reaktion entfernt wurde. Stieg die Kopftemperatur über 68 °C, wurde kein Destillat abgenommen. Im Verlauf der Umesterung fiel eine Menge mₘ an Methanol an (Reaktionszeit t). Während der Reaktion wurden stündlich Proben genommen und diese gaschromatographisch analysiert. Sobald in der GC-Analyse weniger als 0,5 Flächen-% des Monomethylesters zu erkennen waren, wurde das Heizmedium entfernt und der Inhalt des Reaktionskolbens unter Einleiten von Stickstoff auf 80 °C abgekühlt.

Zur Aufarbeitung wurde das Rohprodukt in eine Destillations-Apparatur mit Claisenbrücke und Vakuumteiler überführt. Im Vakuum (ca. 1 mbar) und bei ca. 160 °C bzw. ca. 180 °C Sumpftemperatur (Tributyl- und Tripentylester ca. 160 °C, Tri(2-ethylhexyl)ester und Tri(*^{iso}*nonyl)ester ca. 180 °C) wurde der Überschussalkohol abdestilliert und nachfolgend unter Stickstoffatmosphäre erneut abgekühlt. Vom Kolbeninhalt wurde die Säurezahl bestimmt und dieser dann unter Stickstoffeinperlung (6 I/h) mit der dreifachen stöchiometrischen Menge an Base (10 %-ige wässrige NaOH-Lösung, NaOH von Merck, Reinheit > 99%) bei 80 °C für 15 min gerührt. Anschließend wurden 2 Gew.-% Aktivkohle (Cabot Norit Nederland B.V., CAP Super), bezogen auf die Masse des Kolbeninhaltes, in diesen eingefüllt und 5 Minuten gerührt. Nochmals wurden im Vakuum und bei ca. 160 °C bzw. ca. 180 °C (s.o.) die verbliebenen flüchtigen Anteile mithilfe einer Stickstoffeinleitung abgetrennt, wobei der Stickstoffstrom so eingestellt wurde, dass der Druck nicht über 20 mbar stieg. Sobald der Restalkoholgehalt entsprechend GC-Analyse kleiner als 0,025 Flächen-% war, wurde das resultierende Rohprodukt abgekühlt und über einen Büchnertrichter mit Filterpapier und vorgepresstem Filterkuchen aus Filterhilfsmittel (Perlite Typ D14) mittels Vakuum in eine Saugflasche filtriert.

Es wurden die Menge mₚ des jeweiligen Trialkylcyclohexan-1,2,4-tripropionates (Tc-Ester) mit der in der Tabelle 1 angegebenen Reinheit (%) erhalten.

Besonderheiten der einzelnen Synthesen:
Bei der Herstellung des Tri(2-ethylhexyl)esters, des Tri(*^{iso}*nonyl)esters und des Tri(2-propylheptyl)esters wurde im Laufe der Umesterung (bei 240 °C Sumpftemperatur) schrittweise unter Aufrechterhaltung des Rückflusses Vakuum angelegt. Dabei fiel die Kopftemperatur langsam mit Reduzierung des Druckes.

**Tabelle 1: Details zur Herstellung erfindungsgemäßer Trialkylcyclohexan-1,2,4-tripropionate**

| Bsp. | me (Tc-Ester) [g] / [mol] | Alkohol | mₐ (Alkohol) [g] / [mol] | mₘ (MeOH) [g] / [mol] | Reaktions-zeit t [h] | mₚ / Ausbeute / Reinheit gemäß GC [g] / [%] / [%] |
|---|---|---|---|---|---|---|
| 1* | 171 / 0,5 | "Butanol | 139 / 1,88 | 48 / 1,5 | 6 | 158 / 67 / 98,1 |
| 2* | 514 / 1,5 | *^{iso}*Pentanol | 496/5,6 | 144/4,5 | 3,5 | 617 / 81 / 98,0 |
| 3* | 190 / 0,55 | 2-Ethyl- | 269/2,1 | 53 / 1,65 | 2,5 | 275 / 78 / 98,3 |
| | | hexanol | | | | |
| 4* | 190/0,55 | *^{iso}*Nonanol | 297/2,1 | 53 / 1,65 | 2,5 | 267 / 71 / 98,6 |
| 5* | 240 / 0,7 | 2-Propylheptanol | 415/2,6 | 67 / 2,1 | 8 | 440 / 87 / 98,1 |

| | | | | | | |
|---|---|---|---|---|---|---|
| *erfindungsgemäß "Butanol: Sigma-Aldrich, Reinheit > 99,4 % *^{iso}*Pentanol: Gemisch "Pentanol (Sigma Aldrich, Reinheit >99 %) und 2-Methylbutanol (Sigma Aldrich, Reinheit > 99 %) im Molverhältnis 1:1 2-Ethylhexanol: Sigma-Aldrich, Reinheit > 99 % *^{iso}*Nonanol: Evonik Performance Materials GmbH, Reinheit > 99 % 2-Propylheptanol: Evonik Performance Materials GmbH, Reinheit > 99,5% | | | | | | |

### Beispiele 6 - 10: Herstellung nicht-erfindunqsqemäßer Cyclohexan-1,2,4-tricarbonsäure-trialkylester

In eine Destillations-Apparatur umfassend Tauchrohr, Thermometer und einen Wasserauskreiser mit aufgesetztem Kondensator wurde die Menge mₛ Cyclohexan-1,2,4-tricarbonsäure (Ct-Säure, > 97%) vorgelegt und in der Menge mₐ des Alkohols suspendiert. Die Apparatur wurde mindestens eine Stunde mit Stickstoff (6 I/h) über das Tauchrohr gespült, bevor 0,15 Gew.-% Tetra-"butyltitanat (Sigma Aldrich, Reinheit > 97%), bezogen auf die Masse der Cyclohexan-1,2,4-tricarbonsäure, hinzugegeben wurden. Während einer bis zur Beendigung der Reaktion andauernden Einperlung von Stickstoff (6 I/h) wurde das Gemisch langsam unter Rühren zum Sieden erhitzt. Das anfallende Reaktionswasser wurde kontinuierlich über den Wasserauskreiser aus der Reaktion entfernt. Sobald kein kontinuierlicher Rückfluss mehr vorhanden war, wurde Cyclohexan in der Menge m_{c} als Schleppmittel eingesetzt. Im Verlauf der Veresterung fiel eine Menge m_{w} an Wasser an (Reaktionszeit t). Nach dem Erreichen der theoretischen Menge an Reaktionswasser wurden halbstündlich Proben zur Bestimmung der Säurezahl genommen. Sobald eine Säurezahl von < 0,1 mg KOH/g gemessen wurde, wurde der Inhalt des Reaktionskolbens durch Entfernen der Heizquelle und unter Einleiten von Stickstoff auf 80 °C gekühlt.

Zur Aufarbeitung wurde das Rohprodukt in eine Destillations-Apparatur umfassend Claisenbrücke mit Vakuumteiler überführt. Im Vakuum (ca. 1 mbar) bei ca. 160 °C bzw. ca. 180 °C Sumpftemperatur (Tri(*n*-butyl)ester und Tri(*iso*-pentyl)ester ca. 160 °C, Tri(2-ethylhexyl)ester, Tri(*iso*nonyl)ester und Tri(2-propylheptylester ca. 180 °C) wurde der Überschussalkohol abdestilliert und nachfolgend unter Stickstoffatmosphäre erneut abgekühlt. Vom Kolbeninhalt wurde die Säurezahl bestimmt und dieser dann unter Stickstoffeinperlung (6 I/h) mit der dreifachen stöchiometrischen Menge an Base (10 %-ige wässrige NaOH-Lösung, NaOH von Merck, Reinheit > 99%) bei 80 °C für 15 min gerührt. Anschließend wurden 2 Gew.-% Aktivkohle (Cabot Norit Nederland B.V., CAP Super), bezogen auf die Masse des Kolbeninhaltes, in diesen eingefüllt und 5 Minuten gerührt. Nochmals wurden im Vakuum bei ca. 160 °C bzw. ca. 180 °C (s.o.) die verbliebenen flüchtigen Anteile mithilfe einer Stickstoffeinleitung abgetrennt, wobei der Stickstoffstrom so eingestellt wurde, dass der Druck nicht über 20 mbar stieg. Sobald der Restalkoholgehalt entsprechend GC-Analyse kleiner als 0,025 Flächen-% war, wurde das resultierende Rohprodukt abgekühlt und über einen Büchnertrichter mit Filterpapier und vorgepresstem Filterkuchen aus Filterhilfsmittel (Perlite Typ D14) mittels Vakuum in eine Saugflasche filtriert.

Es wurden die Menge mₚ des jeweiligen Cyclohexan-1,2,4-tricarbonsäure-trialkylesters mit der in der Tabelle 2 angegebenen Reinheit (%) in Prozent erhalten.

Besonderheiten der einzelnen Synthesen:
Herstellung des Tri(*ⁿ*butyl)esters: Gemeinsam mit Tetra-"butyltitanat wurden in diesem Versuch 0,15 Gew.-% Schwefelsäure (Sigma-Aldrich, Reinheit 95-97%), bezogen auf die Masse der Cyclohexan-1,2,4-tricarbonsäure, zugegeben. Des Weiteren wurde der Inhalt des Reaktionskolbens durch Einleiten von Stickstoff auf 80 °C gekühlt, sobald eine Säurezahl von < 1 mg KOH/g (statt < 0,1 mg KOH/g) gemessen wurde.

Herstellung des Tri(*ⁿ*butyl)esters und des Tri(*^{iso}*pentyl)esters: Es wurde zu Beginn der Reaktion lediglich die Hälfte der oben beschriebenen Menge an Tetra-"butyltitanat sowie an dem jeweiligen Alkohol vorgelegt und die restlichen Mengen erst bei Erreichen einer Sumpftemperatur von 240 °C zugegeben.

**Tabelle 2: Details zur Herstellung nicht-erfindungsgemäßer Cyclohexan-1,2,4-tricarbonsäuretrialkylester (Ct-Ester)**

| Bsp. | ms (Ct-Säure) [g] / [mol] | Alkohol | mₐ (Alkohol) [g] / [mol] | m_{w} (H₂O) [g] / [mol] | Reaktions-zeit t / m_{c} (Cyclohexan) [h] / [mL] | Ausbeute / Reinheit gemäß GC [g] / [%] / [%R] |
|---|---|---|---|---|---|---|
| 6 | 184 / 0,85 | "Butanol | 217/2,9 | 46 / 2,6 | 16 / 120 | 240 / 74 / 99,2 |
| 7 | 432 / 2 | *^{iso}*Pentanol | 563/7,5 | 108/6 | 7 / 0 | 583 / 68 / 99,0 |
| 8 | 184 / 0,85 | 2-Ethylhexanol | 382/2,9 | 46 / 2,6 | 3/30 | 339 / 72 / 99,1 |
| 9 | 184 / 0,85 | *^{iso}*Nonanol | 422/2,9 | 46 / 2,6 | 2,5 / 50 | 353 / 70 / 99,5 |
| 10 | 162 / 0,75 | 2-Propylheptanol | 409/2,6 | 41 / 2,3 | 2,5 / 25 | 332 / 69 / 99,4 |

| | | | | | | |
|---|---|---|---|---|---|---|
| *ⁿ*Butanol: Sigma-Aldrich, Reinheit >99,4 % *^{iso}*Pentanol: Gemisch "Pentanol (Sigma Aldrich, Reinheit >99 %) und 2-Methylbutanol (Sigma Aldrich, Reinheit > 99 %) im Molverhältnis 1:1 2-Ethylhexanol: Sigma-Aldrich, Reinheit > 99 % *^{iso}*Nonanol: Evonik Performance Materials GmbH, Reinheit > 99 % 2-Propylheptanol: Evonik Performance Materials GmbH, Reinheit > 99,5 | | | | | | |

### Beispiel 11: Eigenviskosität der Ester aus den Beispielen 1 bis 10

Die Bestimmung der Viskosität erfolgte mittels StabingerViskosimeter (SVM3000 der Fa. Anton Paar), welches eine Abwandlung des klassischen Couette Rotationsviskosimeters darstellt. Die Ester wurden einzeln und blasenfrei gemäß Gebrauchsanweisung in die Messzelle eingespritzt und bei 20 °C vermessen.

Die Eigenviskositäten der Ester sind in der Tabelle 3 aufgelistet.

**Tabelle 3: Eigenviskosität der Ester aus den Beispielen 1 bis 10 bei 20 °C [mPa·s]**

| | Trialkylcyclohexan-1,2,4-tripropionat* | Cyclohexan-1,2,4-tricarbonsäuretrialkylester |
|---|---|---|
| *ⁿ*butyl | 52,3 | 47,4 |
| *^{iso}*pentyl | 79,7 | 69,1 |
| 2-ethylhexyl | 151,8 | 158,0 |
| *^{iso}*nonyl | 168,0 | 163,5 |
| 2-propylheptyl | 217,5 | 222,9 |

| | | |
|---|---|---|
| * erfindungsgemäß | | |

### Beispiel 12: Herstellung von Plastisolen.

Es wurden PVC-Plastisole hergestellt, wie sie beispielsweise zur Fertigung von Deckstrichfilmen für Fußbodenbeläge verwendet werden. Die Angaben in den Plastisolrezepturen sind jeweils in Massenanteilen. Die Rezepturen der Polymerzusammensetzungen sind in Tabelle 4 aufgelistet.

**Tabelle 4: Plastisolrezeptur**

| | phr |
|---|---|
| PVC (Vestolit B 7021 - Ultra; Fa. Vestolit) | 100 |
| Triester(gemisch) aus Beispiel 1*, 2*, 3*, 6, 7 bzw. 8 | 50 |
| epoxidiertes Sojabohnenöl als Co-Stabilisator (Drapex 39, Fa. Galata) | 3 |
| Thermostabilisator auf Ca/Zn-Basis ((Reagens CLX/759/6PF) | 2 |

| | |
|---|---|
| Angaben in phr (phr = parts per hundred parts resin) | |

Zuerst wurden die flüssigen und dann die pulverförmigen Bestandteile in einen PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Nach Einschalten des Rührers wurde die Drehzahl langsam auf ca. 2000 UpM (Umdrehungen pro Minute) erhöht. Währenddessen wurde das Plastisol vorsichtig entlüftet, der Druck musste 20 mbar unterschreiten. Sobald das Plastisol eine Temperatur von ca. 30°C erreicht hatte, wurde die Drehzahl auf ca. 350 UpM gesenkt. Ab jetzt wurde das Plastisol für 9 Minuten bei dieser Drehzahl und einem Druck unterhalb von 20 mbar entlüftet. Damit war sichergestellt, dass die Homogenisierung des Plastisols bei einem definierten Energieeintrag erreicht wurde. Danach wurde das Plastisol sofort für weitere Untersuchungen in einem Klimaschrank auf 25,0 °C temperiert.

### Beispiel 13: Bestimmung des Eindickverhaltens

Die Messung der Viskosität der in Beispiel 12 hergestellten Plastisole wurde mit einem Rheometer Physica MCR 101 (Firma Anton Paar Germany GmbH) mit Hilfe der zugehörigen Software durchgeführt, wobei der Rotationsmodus und das Messsystem CC27 verwendet wurden.

Während der Messung wurden folgende Punkte angesteuert:
- Vorscherung von 100 s⁻¹ für einen Zeitraum von 60 Sekunden, bei der keine Messwerte aufgenommen wurden,
- Scherraten-Abwärtsrampe von 200 s⁻¹ bis 0,1s⁻¹. Es wurden 30 Messpunkte aufgenommen mit einer Messpunktdauer von je 10 Sekunden.

Die Messungen wurden nach einer Lagerung von 2 Stunden, 24 Stunden und 7 Tagen durchgeführt. Zwischen den Messungen wurden die Plastisole bei 25 °C gelagert.

Das Eindickverhalten der Plastisole wurde anhand des prozentualen Viskositätsanstiegs nach 24 Stunden und nach 7 Tagen, bezogen auf die Viskositätswerte nach 2 Stunden, bei einer Scherrate von 1, 10 und 100 s⁻¹ bestimmt.

**Tabelle 5: Eindickverhalten der Plastisole aus Beispiel 12 bei 1 s⁻¹**

| | | Viskosität [Pa·s] | Viskosität [Pa·s] | Eindicken [%] | Viskosität [Pa·s] | Eindicken [%] |
|---|---|---|---|---|---|---|
| | | nach 2 h | nach 24 h | | nach 7 Tagen | |
| Trialkylcyclo-hexan-1,2,4-tripropionat | *ⁿ*butyl* | 2,53 | 3,3 | 30,4 | 4,77 | 88,5 |
| | *^{iso}*pentyl* | 2,64 | 3,15 | 19,3 | 3,97 | 50,4 |
| | 2-ethylhexyl* | 3,15 | 3,36 | 6,7 | 3,66 | 16,2 |
| Cyclohexan-1,2,4-tricarbonsäuretrialkylester | *ⁿ*butyl | 6,23 | 11,2 | 79,8 | 26,5 | 325,4 |
| | *^{iso}*pentyl | 3,62 | 5,04 | 39,2 | 7,63 | 110,8 |
| | 2-ethylhexyl | 3,92 | 4,44 | 13,3 | 5,28 | 34,7 |

| | | | | | | |
|---|---|---|---|---|---|---|
| * erfindungsgemäß | | | | | | |

**Tabelle 6: Eindickverhalten der Plastisole aus Beispiel 12 bei 10 s⁻¹**

| | | Viskosität [Pa·s] | Viskosität [Pa·s] | Eindicken [%] | Viskosität [Pa·s] | Eindicken [%] |
|---|---|---|---|---|---|---|
| | | nach 2 h | nach 24 h | | nach 7 Tagen | |
| Trialkylcyclo-hexan-1,2,4-tripropionat | *ⁿ*butyl* | 2,28 | 2,94 | 28,9 | 4,11 | 80,3 |
| | *^{iso}*pentyl* | 2,51 | 2,99 | 19,1 | 3,71 | 47,8 |
| | 2-ethylhexyl* | 3,26 | 3,47 | 6,4 | 3,76 | 15,3 |
| Cyclohexan-1,2,4-tricarbonsäuretrialkylester | *ⁿ*butyl | 5,44 | 9,42 | 73,2 | 20,9 | 284,2 |
| | *^{iso}*pentyl | 3,32 | 4,37 | 31,6 | 6,36 | 91,6 |
| | 2-ethylhexyl | 4,05 | 4,52 | 11,6 | 5,27 | 30,1 |

| | | | | | | |
|---|---|---|---|---|---|---|
| * erfindungsgemäß | | | | | | |

**Tabelle 7: Eindickverhalten der Plastisole aus Beispiel 12 bei 100 s⁻¹**

| | | Viskosität [Pa·s] | Viskosität [Pa·s] | Eindicken [%] | Viskosität [Pa·s] | Eindicken [%] |
|---|---|---|---|---|---|---|
| | | nach 2 h | nach 24 h | | nach 7 Tagen | |
| Trialkylcyclo-hexan-1,2,4-tripropionat | *ⁿ*butyl* | 3,11 | 3,98 | 28 | 5,41 | 74 |
| | *^{iso}*pentyl* | 3,7 | 4,34 | 17,3 | 5,27 | 42,4 |
| | 2-ethylhexyl* | 5,77 | 5,92 | 2,6 | 6,19 | 7,3 |
| Cyclohexan-1,2,4-tricarbonsäuretrialkylester | *ⁿ*butyl | 6,71 | 10,8 | 61 | 21 | 213 |
| | *^{iso}*pentyl | 4,66 | 5,88 | 26,2 | 8,1 | 73,8 |
| | 2-ethylhexyl | 6,61 | 7,09 | 7,3 | 7,97 | 20,6 |

| | | | | | | |
|---|---|---|---|---|---|---|
| * erfindungsgemäß | | | | | | |

Die Plastisolviskosität ist für erfindungsgemäße Trialkylcyclo-hexan-1,2,4-tripropionate geringer als die Plastisolviskosität der Vergleichsester. Zudem steigt die Viskosität der Trialkyl-cyclohexan-1,2,4-tripropionate mit der Zeit weniger stark an als bei den Vergleichsestern. Aufgrund dieser vorteilhaften Eigenschaft können die erfindungsgemäßen Triester auch noch nach längerem Stehen ohne Zugabe von die Viskosität senkenden Additiven verwendet werden, was neben dem Einsatz dieser Additive auch den mit deren Einsatz verbundenen Arbeits- und Zeitaufwand einspart.

### Beispiel 14: Herstellung von Folien

Die im Beispiel 12 hergestellten Plastisole wurden jeweils zu 1 mm dicken Folien verarbeitet.

Dazu wurde zunächst Hochglanztrennpapier (Fa. Sappi, Italien) auf eine Größe von 30 x 44 cm zugeschnitten und im Spannrahmen der Streicheinrichtung LTSV für den Mathis-Ofen eingelegt. Danach wurde der Spannrahmen auf den Führungsrahmen aufgelegt, der Mathis-Ofen (Typ LTF) auf 200 °C eingestellt und der Rahmen nach Erreichen dieser Temperatur 15 Sekunden vorgewärmt. Danach wurde die Rakel in die Einspannvorrichtung gelegt und der Rakelspalt über Vorversuche so eingestellt, dass die Foliendicke nach Abschluss der Gelierung 1 mm (+/- 0,05 mm) betrug. Auf den vorderen Rand des Papiers wurde ein Klebestreifen angebracht, um überschüssiges Plastisol aufzufangen. Danach wurde das Plastisol vor der Rakel aufgetragen und diese durch Ziehen des Führungsrahmens mit der Rakel über das eingespannte Trennpapier verstrichen (3 m/min Geschwindigkeit). Danach wurde die Rakel herausgenommen und der Klebestreifen mit dem überschüssigen Plastisol abgenommen. Anschließend wurde der Spannrahmen in den Ofen eingefahren. Nach der Gelierung (2 Minuten bei 200 °C) wurde der Rahmen wieder aus dem Ofen herausgefahren und nach Abkühlen die Folie von dem Papier abgezogen.

### Beispiel 15: Massenverlust der Folien

Pro Rezeptur wurden je 6 Schulterstäbe (Typ S2 gemäß DIN 53504) aus dem Beispiel 14 über Nacht unter Normklima (23 °C, 50 % relative Luftfeuchtigkeit) konditioniert und anschließend gewogen. Anschließend wurden die Schulterstäbe hängend in einem umluftbetriebenen Wärmeschrank über einer Schale (28 x 20 x 6 cm) gefüllt mit Aktivkohle mit einem Mindestabstand von 20 mm bei 80 °C gelagert. Nach 7 beziehungsweise 14 Tagen wurden die Schulterstäbe entnommen, über Nacht in einem Exsikkator gelagert und anschließend gewogen. Der Massenverlust wurde über Differenzbildung für jeden einzelnen Schulterstab bestimmt. In der Tabelle 8 sind die Mittelwerte für den Massenverlust von jeweils sechs Einzelmessungen pro Rezeptur in Prozent angegeben.

**Tabelle 8: Massenverlust von Folien an der Luft (80 °C)**

| | Trialkylcyclohexan-1,2,4-tripropionat* | | Cyclohexan-1,2,4-tricarbonsäuretrialkylester | |
|---|---|---|---|---|
| | nach 7 d | nach 14 d | nach 7 d | nach 14 d |
| *ⁿ*butyl | 0,8 % | 0,9 % | 3,4 % | 6,1 % |
| *^{iso}*pentyl | 0,7 % | 0,8 % | 1,5 % | 2,4 % |
| 2-ethylhexyl | 0,6 % | 0,7 % | 0,8 % | 0,9 % |

| | | | | |
|---|---|---|---|---|
| * erfindungsgemäß | | | | |

Der Massenverlust von Folien enthaltend erfindungsgemäße Triester ist geringer als der von Folien, welche die entsprechenden Cyclohexan-1,2,4-tricarbonsäuretrialkylester enthalten.

### Beispiel 16: Glasübergangstemperatur der Folien

Die Glasüberganstemperatur wurde mit Hilfe von DMTA-Messungen gemäß DIN 65583 mit einem Rheometer der Firma Anton Paar vom Typ MCR 302 bestimmt. Unter konstanten dynamischmechanischen Bedingungen (1 Hz, Deformation 0,3 %) wurden die viskoelastischen Eigenschaften der Folien in Abhängigkeit der Temperatur (Temperaturrampe von -100 bis +50 °C) erfasst und das Speichermodul, das Verlustmodul und der Verlustfaktor bestimmt. Das Maximum des Verlustmoduls wird dabei als Glasübergangstemperatur ausgewertet. Die nachfolgende Tabelle zeigt jeweils den Mittelwert aus einer Doppelbestimmung.

**Tabelle 9: Glasübergangstemperaturen T_{g} der Folien in °C**

| | Trialkylcyclohexan-1,2,4-tripropionat* | Cyclohexan-1,2,4-tricarbonsäuretrialkylester |
|---|---|---|
| *ⁿ*butyl | -27 | -15 |
| *^{iso}*pentyl | -28 | -18 |
| 2-ethylhexyl | -37 | -24 |

| | | |
|---|---|---|
| * erfindungsgemäß | | |

Wie aus den niedrigeren Glasübergangstemperaturen ersichtlich, ist die Tieftemperaturflexibilität bei erfindungsgemäßen Estern deutlich verbessert gegenüber den Vergleichsverbindungen.

### Beispiel 17: Herstellung von Drvblends, Walzfellen und Pressplatten

Die für die nachfolgenden Beispiele benötigten Prüfkörper werden durch Trockenvermischen (Dryblendherstellung), Kalandrieren (Walzen) und Pressen der folgenden Rezepturen hergestellt:

**Tabelle 10: Dryblend-Rezeptur**

| | phr |
|---|---|
| PVC (Inovyn 271 PC; Firma Inovyn) | 100 |
| Triester(gemische) aus Beispiel 3*, 4*, 5*, 8, 9, 10, Tri(2-ethylhexyl)trimellitat oder Tri(*^{iso}*nonyl)trimellitat | 50 |
| Füllstoff Kreide (OMYA BSH) | 20 |
| Thermostabilisator (Baeropan MC 8890/KA/2/MC) | 10 |

| | |
|---|---|
| phr: parts per hundred parts resin Tri(2-ethylhexyl)trimellitat: Eastman Chemical Company, Reinheit > 99 % Tri(*^{iso}*nonyl)trimellitat: UPC Technology, Taiwan, Reinheit > 98 % | |

Mit Trockenmischungen, die als Dryblends bezeichnet werden, lassen sich zum Beispiel nach thermoplastischer Verarbeitung (z.B. Kalandrieren oder Extrudieren) Kabel- und Drahtisolierungen, Schläuche oder Fußböden und Dachbahnen herstellen.

Die Herstellung der Dryblends erfolgte in einem Brabender Planetenmischer.

Über die Software "Winmix" wurden folgende Parameter am Brabender Planetenmischer eingestellt.

| | |
|---|---|
| Drehzahlprogramm: | Aktiv |
| Profil: | Drehzahl 50 U/min; Haltezeit: 9 min; Anstiegszeit (der Drehzahl): 1 min; Drehzahl 100 U/min; Haltezeit: 20 min |
| Temperatur: | 88 °C |
| Messbereich: | 2 Nm |
| Dämpfung: | 3 |

Die Temperatur im Mischbehälter betrug nach einstündiger Temperierzeit 88 °C. Nachdem der Planetenmischer eine Eigenkalibrierung durchgeführt hatte, wurden die festen Bestandteile (PVC, Stabilisator), welche vorher auf einer Analysenwaage in vierfacher Menge (vierfache Menge in g bezogen auf die Tabelle 10 in phr) in einen PE-Becher einwogen wurden, dem Mischgefäß über einen Feststofftrichter und den vorhandenen Einfüllstutzen am Brabender Mischgefäß zugeführt. Das Programm wurde gestartet und die Pulvermischung wurde 9 Minuten im Mischgefäß gerührt und temperiert, ehe die flüssigen Bestandteile, welche ebenfalls in vierfacher Menge auf der Waage in einem PE-Becher ausgewogen wurden, über einen Flüssigkeitstrichter und den vorhandenen Einfüllstutzen am Brabender Mischgefäß, zugeführt wurden. Die Mischung wurde weitere 20 Minuten im Planetenmischer gerührt. Nach Beendigung des Programms wurde die fertige Trockenmischung (Dryblend) entnommen.

Aus diesen Dryblends wurden Walzfelle hergestellt. Die Herstellung der Walzfelle erfolgte auf einem Kalander W150 AP der Firma Collin. Der Kalander der Firma Collin besitzt einen automatischen Probenumleger und wird über einen zusätzlichen Ölthermostaten temperiert. Die Steuerung erfolgte über Software der Fa. Collin.

Es wurde ein fünfstufiges Programm zur Herstellung des Walzfelles verwendet:

| Stufe | Bezeichnung | Temp. [°C] | Dauer [s] | Spaltbreite [mm] | Drehzahl [Upm] |
|---|---|---|---|---|---|
| 1 | Plastifizierung des Dryblends | 165 | 60 | 0,2 | 5 |
| 2 | Vergrößerung des Spalts | 165 | 30 | 0,5 | 20 |
| 3 | Aktivierung des Probenumlegers | 165 | 170 | 0,5 | 20 |
| 4 | Walzfelloptimierung | 165 | 30 | 0,5 | 25 |
| 5 | Walzfellabnahme | 165 | 60 | 0,5 | 7 |

Nach dem Erreichen der Walzentemperatur wurde der Walzenspalt kalibriert. Zum Start der Messung wurde der Walzenspalt auf 0,2 mm eingestellt. Jeweils 160 g eines Dryblends wurden eingewogen und bei stehenden Walzen in den Walzenspalt gegeben. Das Programm wurde gestartet.

Die Pressplatten wurden mit einer Laborpresse der Firma Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke übereinander in den 15 x 15 cm großen Pressrahmen aus Edelstahl gelegt.

Es wurde dreistufiges Programm zur Herstellung der Pressplatten verwendet:

| Stufe | Bezeichnung | Temp. [°C] | Druck [bar] | Dauer [s] |
|---|---|---|---|---|
| 1 | Vorpressen | 170 | 5 | 60 |
| 2 | Pressen | 170 | 200 | 200 |
| 3 | Abkühlen | 40 | 200 | 200 |

### Beispiel 18: Glasüberqanqstemperatur der Pressplatten

Die Glasüberganstemperatur wurde mit Hilfe von DMTA-Messungen gemäß DIN 65583 mit einem Rheometer der Firma Anton Paar vom Typ MCR 302 bestimmt. Unter konstanten dynamischmechanischen Bedingungen (1 Hz, Deformation 0,3 %) wurden die viskoelastischen Eigenschaften der Folien in Abhängigkeit der Temperatur (Temperaturrampe von -100 bis +50 °C) erfasst und der Speichermodul, das Verlustmodul und der Verlustfaktor bestimmt. Das Maximum des Verlustmoduls wird dabei als Glasübergangstemperatur ausgewertet. Die nachfolgende Tabelle zeigt jeweils den Mittelwert aus einer Doppelbestimmung.

**Tabelle 11: Glasübergangstemperaturen T_{g} der Pressplatten in °C**

| | Trialkylcyclohexan-1,2,4-tripropionat* | Cyclohexan-1,2,4-tricarbonsäuretrialkylester | Trialkyltrimellitat |
|---|---|---|---|
| 2-ethylhexyl | -31 | -16 | -18 |
| *^{iso}*nonyl | -38 | -24 | -27 |
| 2-propylheptyl | -57 | -35 | - |

| | | | |
|---|---|---|---|
| * erfindungsgemäß | | | |

Wie aus den niedrigeren Glasübergangstemperaturen ersichtlich ist die Tieftemperaturflexibilität bei erfindungsgemäßen Estern höher als bei den Vergleichsverbindungen. Eine gute Tieftemperaturflexibilität ist insbesondere relevant für Außenanwendungen.

### Beispiel 19: Massenverlust der Pressplatten

Aus den Pressplatten des Beispiels 17 wurden pro Rezeptur je 6 Prüfkörper in Form von Zugstäben des Typs S2 ausgestanzt, über Nacht in einem Exsikkator konditioniert und anschließend gewogen. Anschließend wurden die Prüfkörper hängend in einem umluftbetriebenen Wärmeschrank über einer Schale (28 x 20 x 6 cm) gefüllt mit Aktivkohle mit einem Mindestabstand von 20 mm bei 135 °C gelagert. Nach 14 Tagen wurden die Prüfkörper entnommen, über Nacht unter Normklima gelagert und anschließend gewogen. Die Differenz der für den jeweiligen Prüfkörper bestimmten Massen stellt den Massenverlust dar. In der Tabelle 12 sind die Mittelwerte von jeweils drei Einzelmessungen pro Rezeptur in Prozent angegeben.

**Tabelle 12: Massenverlust von Pressplatten an der Luft (nach 14 tägiger Lagerung bei 135 °C) in Massen%**

| | Trialkylcyclohexan-1,2,4-tripropionat* | Cyclohexan-1,2,4-tricarbonsäuretrialkylester | Trialkyltrimellitat |
|---|---|---|---|
| 2-ethylhexyl | -1,7 | -11,5 | -7 |
| *^{iso}*nonyl | -1,3 | -2,9 | -1,8 |
| 2-propylheptyl | -1,7 | -2,4 | - |

| | | | |
|---|---|---|---|
| * erfindungsgemäß | | | |

Der Massenverlust von Pressplatten enthaltend erfindungsgemäße Triester ist geringer als der von Folien, welche die entsprechenden Cyclohexan-1,2,4-tricarbonsäuretrialkylester oder die entsprechenden Trialkyltrimellitate enthalten.

## Patentansprüche

1. Triester der Cyclohexantripropionsäure, in welchen die drei Alkoholteile der drei Estergruppen jeweils 2, 3, 4, 5, 6, 7, 8, 9, 10, 11 oder 12 Kohlenstoff-Atome umfassen.

2. Triester der Cyclohexantripropionsäure gemäß Anspruch 1, **dadurch gekennzeichnet, dass** es sich um Triester der Cyclohexan-1,2,4-tripropionsäure oder um Triester der Cyclohexan-1,3,5-tripropionsäure handelt.

3. Triester der Cyclohexantripropionsäure gemäß Anspruch 1 oder 2, **dadurch gekennzeichnet, dass** die Alkoholteile neben dem Sauerstoff der Esterfunktion keine weiteren Heteroatome und keine Mehrfachbindungen enthalten.

4. Triester der Cyclohexantripropionsäure gemäß einem der Ansprüche 1 bis 3, **dadurch gekennzeichnet, dass** sämtliche in einem Molekül enthaltenen Alkoholteile identische Summenformeln und gleichzeitig identische oder unterschiedliche Konstitutionsformeln aufweisen.

5. Mischung von mindestens zwei Triestern der Cyclohexantripropionsäure gemäß einem der Ansprüche 1 bis 4.

6. Mischung gemäß Anspruch 5, **dadurch gekennzeichnet, dass** sich die mindestens zwei Triester der Cyclohexantripropionsäure in ihren Summenformeln und/oder in ihren Konstitutionsformeln unterscheiden.

7. Triester der Cyclohexantripropionsäure gemäß einem der Ansprüche 1 bis 4 oder Mischung gemäß Anspruch 5 oder 6, **dadurch gekennzeichnet, dass** er/sie ausgewählt ist aus der Gruppe bestehend aus:
Tri(*ⁿ*butyl)-cyclohexan-1,2,4-tripropionat, Tri(methylpropyl)-cyclohexan-1,2,4-tripropionat, Tri(*ⁿ*pentyl)-cyclohexan-1,2,4-tripropionat, Tri(*^{iso}*pentyl)-cyclohexan-1,2,4-tripropionat, Tri(2-methylbutyl)-cyclohexan-1,2,4-tripropionat, Tri(3-methylbutyl)-cyclohexan-1,2,4-tripropionat, Tri(*ⁿ*hexyl)-cyclohexan-1,2,4-tripropionat, Tri(*^{iso}*hexyl)-cyclohexan-1,2,4-tripropionat, Tri(*ⁿ*heptyl)-cyclohexan-1,2,4-tripropionat, Tri(*^{iso}*heptyl)-cyclohexan-1,2,4-tripropionat, Tri(*ⁿ*octyl)-cyclohexan-1,2,4-tripropionat, Tri(*^{iso}*octyl)-cyclohexan-1,2,4-tripropionat, Tri(2-ethylhexyl)-cyclohexan-1,2,4-tripropionat, Tri(*ⁿ*nonyl)-cyclohexan-1,2,4-tripropionat, Tri(*^{iso}*nonyl)-cyclohexan-1,2,4-tripropionat, Tri(*ⁿ*decyl)-cyclohexan-1,2,4-tripropionat, Tri(*^{iso}*decyl)-cyclohexan-1,2,4-tripropionat, Tri(2-propylheptyl)-cyclohexan-1,2,4-tripropionat,
Tri(*ⁿ*butyl)-cyclohexan-1,3,5-tripropionat, Tri(methylpropyl)-cyclohexan-1,3,5-tripropionat, Tri(*ⁿ*pentyl)-cyclohexan-1,3,5-tripropionat, Tri(*^{iso}*pentyl)-cyclohexan-1,3,5-tripropionat, Tri(2-methylbutyl)-cyclohexan-1,3,5-tripropionat, Tri(3-methylbutyl)-cyclohexan-1,3,5-tripropionat, Tri(*ⁿ*hexyl)-cyclohexan-1,3,5-tripropionat, Tri(*^{iso}*hexyl)-cyclohexan-1,3,5-tripropionat, Tri(*ⁿ*heptyl)-cyclohexan-1,3,5-tripropionat, Tri(*^{iso}*heptyl)-cyclohexan-1,3,5-tripropionat, Tri(*ⁿ*octyl)-cyclohexan-1,3,5-tripropionat, Tri(*^{iso}*octyl)-cyclohexan-1,3,5-tripropionat, Tri(2-ethylhexyl)-cyclohexan-1,3,5-tripropionat, Tri(*ⁿ*nonyl)-cyclohexan-1,3,5-tripropionat, Tri(*^{iso}*nonyl)-cyclohexan-1,3,5-tripropionat, Tri(*ⁿ*decyl)-cyclohexan-1,3,5-tripropionat, Tri(*^{iso}*decyl)-cyclohexan-1,3,5-tripropionat und Tri(2-propylheptyl)-cyclohexan-1,3,5-tripropionat.

8. Weichmacher für Polymere umfassend einen Triester der Cyclohexantripropionsäure gemäß einem der Ansprüche 1 bis 4 und 7 oder eine Mischung gemäß einem der Ansprüche 5 bis 7 und optional mindestens eine weitere Polymer-weichmachende Verbindung.

9. Zusammensetzung, enthaltend einen Triester der Cyclohexantripropionsäure gemäß einem der Ansprüche 1 bis 4 und 7 oder eine Mischung gemäß einem der Ansprüche 5 bis 7 oder einen Weichmacher gemäß Anspruch 8 und ein oder mehrere Polymere.

10. Zusammensetzung gemäß Anspruch 9, **dadurch gekennzeichnet, dass** mindestens ein Polymer ausgewählt ist aus der Gruppe bestehend aus Polyvinylchlorid, Polyalkylmethacrylat (PAMA), Polyvinylbutyral (PVB), Polyurethan, Polysulfide, Polymilchsäure (PLA), Polyhydroxybutyral (PHB), Nitrocellulose und Co-Polymeren von Vinylchlorid mit Vinylacetat oder mit Butylacrylat.

11. Zusammensetzung gemäß einem der Ansprüche 9 oder 10, **dadurch gekennzeichnet, dass** es Bestandteil ist eines Klebstoffs, einer Dichtungsmasse, einer Beschichtungsmasse, eines Lacks, einer Farbe, eines Plastisols, eines Dryblends, eines Schaums, eines Kunstleders, eines Fußbodenbelags, insbesondere dessen Deck- oder Schaumschicht, einer Dachbahn, eines Unterbodenschutzes, einer Gewebebeschichtung, eines Kabels, einer Drahtisolierung, eines Schlauchs, eines Extrusionsartikels, einer Folie, eines Gegenstands im Automobilinnenbereich, einer Tapete, einer Tinte, eines Spielzeugs, einer Kontaktfolie, einer Lebensmittelverpackung oder eines medizinischen Artikels, insbesondere eines Schlauchs oder eines Blutbeutels.

12. Verwendung eines Triesters der Cyclohexantripropionsäure gemäß einem der Ansprüche 1 bis 4 und 7 oder einer Mischung gemäß einem der Ansprüche 5 bis 7 als Weichmacher für Polymere, insbesondere für PVC.

13. Verwendung gemäß Anspruch 12, **dadurch gekennzeichnet, dass** ein Triester der Cyclohexantripropionsäure, dessen Alkoholteile jeweils 7 bis 12 Kohlenstoff-Atome umfassen, als Polymer-weichmachende Verbindung in Hochtemperaturanwendungen eingesetzt wird.

14. Verwendung gemäß Anspruch 12, **dadurch gekennzeichnet, dass** ein Triester der Cyclohexantripropionsäure, dessen Alkoholteile jeweils 2 bis 9 Kohlenstoff-Atome umfassen, als Polymer-weichmachende Verbindung in Plastisol-Anwendungen eingesetzt wird.

15. Herstellung von Triestern der Cyclohexantripropionsäure gemäß einem der Ansprüche 1 bis 4 und 7 durch
- Kernhydrierung der korrespondierenden Triester der Benzoltripropionsäure,
- Umesterung vom Trialkylester der Cyclohexantripropionsäure mit mindestens einem Alkohol umfassend 2, 3, 4, 5, 6, 7, 8, 9, 10, 11 oder 12 Kohlenstoff-Atome, wobei der Alkohol des einzubauenden Alkoholteils des Trialkylesters höher siedet als der Alkohol des Alkoholteils, der im Rahmen der Umesterung ersetzt werden soll,
- Veresterung von Cyclohexantripropionsäure mit mindestens einem Alkohol umfassend 2, 3, 4, 5, 6, 7, 8, 9, 10, 11 oder 12 Kohlenstoff-Atome oder
- Alkoxycarbonylierung von Trivinylcyclohexan mit mindestens einem Alkohol umfassend 2, 3, 4, 5, 6, 7, 8, 9, 10, 11 oder 12 Kohlenstoff-Atome.

## Claims

1. Triester of cyclohexanetripropionic acid, in which the three alcohol moieties of the three ester groups each comprise 2, 3, 4, 5, 6, 7, 8, 9, 10, 11 or 12 carbon atoms.

2. Triester of cyclohexanetripropionic acid according to Claim 1, **characterized in that** it is a triester of cyclohexane-1,2,4-tripropionic acid or a triester of cyclohexane-1,3,5-tripropionic acid.

3. Triester of cyclohexanetripropionic acid according to Claim 1 or 2, **characterized in that** the alcohol moieties, besides the oxygen of the ester function, do not comprise any other heteroatoms, and contain no multiple bonds.

4. Triester of cyclohexanetripropionic acid according to any of Claims 1 to 3, **characterized in that** all alcohol moieties present in one molecule have identical empirical formulae and at the same time identical or different structural formulae.

5. Mixture of at least two triesters of cyclohexanetripropionic acid according to any of Claims 1 to 4.

6. Mixture according to Claim 5, **characterized in that** the at least two triesters of cyclohexanetripropionic acid differ in their empirical formulae and/or in their structural formulae.

7. Triester of cyclohexanetripropionic acid according to any of Claims 1 to 4 or mixture according to Claim 5 or 6, **characterized in that** it is selected from the group consisting of:
tri(*ⁿ*butyl) cyclohexane-1,2,4-tripropionate, tri(methylpropyl) cyclohexane-1,2,4-tripropionate, tri(*ⁿ*pentyl) cyclohexane-1,2,4-tripropionate, tri (*^{iso}*pentyl) cyclohexane-1,2,4-tripropionate, tri(2-methylbutyl) cyclohexane-1,2,4-tripropionate, tri(3-methylbutyl) cyclohexane-1,2,4-tripropionate, tri(*ⁿ*hexyl) cyclohexane-1,2,4-tripropionate, tri(*^{iso}*hexyl) cyclohexane-1,2,4-tripropionate, tri(*ⁿ*heptyl) cyclohexane-1,2,4-tripropionate, tri(*^{iso}*heptyl) cyclohexane-1,2,4-tripropionate, tri(*ⁿ*octyl) cyclohexane-1,2,4-tripropionate, tri(*^{iso}*octyl) cyclohexane-1,2,4-tripropionate, tri(2-ethylhexyl) cyclohexane-1,2,4-tripropionate, tri(*ⁿ*nonyl) cyclohexane-1,2,4-tripropionate, tri(*^{iso}*nonyl) cyclohexane-1,2,4-tripropionate, tri(*ⁿ*decyl) cyclohexane-1,2,4-tripropionate, tri(*^{iso}*decyl) cyclohexane-1,2,4-tripropionate, tri(2-propylheptyl) cyclohexane-1,2,4-tripropionate,
tri(*ⁿ*butyl) cyclohexane-1,3,5-tripropionate, tri(methylpropyl) cyclohexane-1,3,5-tripropionate, tri(*ⁿ*pentyl) cyclohexane-1,3,5-tripropionate, tri (*^{iso}*pentyl) cyclohexane-1,3,5-tripropionate, tri(2-methylbutyl) cyclohexane-1,3,5-tripropionate, tri(3-methylbutyl) cyclohexane-1,3,5-tripropionate, tri(*ⁿ*hexyl) cyclohexane-1,3,5-tripropionate, tri(*^{iso}*hexyl) cyclohexane-1,3,5-tripropionate, tri(*ⁿ*heptyl) cyclohexane-1,3,5-tripropionate, tri(*^{iso}*heptyl) cyclohexane-1,3,5-tripropionate, tri(*ⁿ*octyl) cyclohexane-1,3,5-tripropionate, tri(*^{iso}*octyl) cyclohexane-1,3,5-tripropionate, tri(2-ethylhexyl) cyclohexane-1,3,5-tripropionate, tri(*ⁿ*nonyl) cyclohexane-1,3,5-tripropionate, tri(*^{iso}*nonyl) cyclohexane-1,3,5-tripropionate, tri(*ⁿ*decyl) cyclohexane-1,3,5-tripropionate, tri(*^{iso}*decyl) cyclohexane-1,3,5-tripropionate and tri(2-propylheptyl) cyclohexane-1,3,5-tripropionate.

8. Plasticizer for polymers comprising a triester of cyclohexanetripropionic acid according to any of Claims 1 to 4 and 7 or a mixture according to any of Claims 5 to 7 and optionally at least one other polymer-plasticizing compound.

9. Composition comprising a triester of cyclohexanetripropionic acid according to any of Claims 1 to 4 and 7 or a mixture according to any of Claims 5 to 7 or a plasticizer according to Claim 8 and one or more polymers.

10. Composition according to Claim 9, **characterized in that** at least one polymer is selected from the group consisting of polyvinyl chloride, polyalkyl methacrylate (PAMA), polyvinyl butyral (PVB), polyurethane, polysulfide, polylactic acid (PLA), polyhydroxybutyral (PHB), nitrocellulose and copolymers of vinyl chloride with vinyl acetate or with butyl acrylate.

11. Composition according to either of Claims 9 or 10, **characterized in that** said composition is a constituent of an adhesive, of a sealing compound, of a coating composition, of a lacquer, of a paint, of a plastisol, of a dryblend, of a foam, of a synthetic leather, of a floor covering, particularly the top layer or foam layer thereof, of a roofing membrane, of an underbody protection, of a fabric coating, of a cable, of a wire insulation, of a hose, of an extruded article, of a film, of an article in the automotive interior sector, of a wallpaper, of an ink, of a toy, of a contact sheet, of a food packaging or of a medical article, especially of a tube or of a blood bag.

12. Use of a triester of cyclohexanetripropionic acid according to any of Claims 1 to 4 and 7 or a mixture according to any of Claims 5 to 7 as plasticizer for polymers, especially for PVC.

13. Use according to Claim 12, **characterized in that** a triester of cyclohexanetripropionic acid, the alcohol moieties of which each comprise 7 to 12 carbon atoms, is used as polymer-plasticizing compound in high temperature applications.

14. Use according to Claim 12, **characterized in that** a triester of cyclohexanetripropionic acid, the alcohol moieties of which each comprise 2 to 9 carbon atoms, is used as polymer-plasticizing compound in plastisol applications.

15. Preparation of triesters of cyclohexanetripropionic acid according to any of Claims 1 to 4 and 7 by
- ring hydrogenation of the corresponding triester of benzenetripropionic acid,
- transesterification of the trialkyl ester of the cyclohexanetripropionic acid with at least one alcohol comprising 2, 3, 4, 5, 6, 7, 8, 9, 10, 11 or 12 carbon atoms, wherein the alcohol of the alcohol moiety of the trialkyl ester to be incorporated is higher boiling than the alcohol of the alcohol moiety which is to be replaced in the context of the transesterification,
- esterification of cyclohexanetripropionic acid with at least one alcohol comprising 2, 3, 4, 5, 6, 7, 8, 9, 10, 11 or 12 carbon atoms or
- alkoxycarbonylation of trivinylcyclohexane with at least one alcohol comprising 2, 3, 4, 5, 6, 7, 8, 9, 10, 11 or 12 carbon atoms.

## Revendications

1. Triesters de l'acide cyclohexanetripropionique, dans lesquels les trois parties alcool des trois groupes ester comprennent à chaque fois 2, 3, 4, 5, 6, 7, 8, 9, 10, 11 ou 12 atomes de carbone.

2. Triesters de l'acide cyclohexanetripropionique selon la revendication 1, **caractérisés en ce qu'**il s'agit de triesters de l'acide cyclohexane-1,2,4-tripropionique ou de triesters de l'acide cyclohexane-1,3,5-tripropionique.

3. Triesters de l'acide cyclohexanetripropionique selon la revendication 1 ou 2, **caractérisés en ce que** les parties alcool ne contiennent, outre l'oxygène de la fonction ester, pas d'autres hétéroatomes et pas de liaisons multiples.

4. Triesters de l'acide cyclohexanetripropionique selon l'une quelconque des revendications 1 à 3, **caractérisés en ce que** l'ensemble des parties alcool contenues dans une molécule présentent des formules brutes identiques et simultanément des formules constitutives identiques ou différentes.

5. Mélange d'au moins deux triesters de l'acide cyclohexanetripropionique selon l'une quelconque des revendications 1 à 4.

6. Mélange selon la revendication 5, **caractérisé en ce que** lesdits au moins deux triesters de l'acide cyclohexanetripropionique se distinguent par leurs formules brutes et/ou par leurs formules constitutives.

7. Triesters de l'acide cyclohexanetripropionique selon l'une quelconque des revendications 1 à 4 ou mélange selon la revendication 5 ou 6, **caractérisés en ce qu'**ils sont choisis dans le groupe constitué par :
le cyclohexane-1,2,4-tripropionate de tri(ⁿbutyle), le cyclohexane-1,2,4-tripropionate de tri(méthylpropyle), le cyclohexane-1,2,4-tripropionate de tri(ⁿpentyle), le cyclohexane-1,2,4-tripropionate de tri(^{iso}pentyle), le cyclohexane-1,2,4-tripropionate de tri(2-méthylbutyle), le cyclohexane-1,2,4-tripropionate de tri(3-méthylbutyle), le cyclohexane-1,2,4-tripropionate de tri(ⁿhexyle) , le cyclohexane-1,2,4-tripropionate de tri(^{iso}hexyle) , le cyclohexane-1,2,4-tripropionate de tri(ⁿheptyle), le cyclohexane-1,2,4-tripropionate de tri(^{iso}heptyle), le cyclohexane-1,2,4-tripropionate de tri(ⁿoctyle), le cyclohexane-1,2,4-tripropionate de tri(^{iso}octyle) , le cyclohexane-1,2,4-tripropionate de tri(2-éthylhexyle), le cyclohexane-1,2,4-tripropionate de tri(ⁿnonyle), le cyclohexane-1,2,4-tripropionate de tri(^{iso}nonyle) , le cyclohexane-1,2,4-tripropionate de tri(ⁿdécyle), le cyclohexane-1,2,4-tripropionate de tri(^{iso}décyle), le cyclohexane-1,2,4-tripropionate de tri(2-propylheptyle),
le cyclohexane-1,3,5-tripropionate de tri(ⁿbutyle), le cyclohexane-1,3,5-tripropionate de tri(méthylpropyle), le cyclohexane-1,3,5-tripropionate de tri(ⁿpentyle), le cyclohexane-1,3,5-tripropionate de tri(^{iso}pentyle), le cyclohexane-1,3,5-tripropionate de tri(2-méthylbutyle), le cyclohexane-1,3,5-tripropionate de tri(3-méthylbutyle), le cyclohexane-1,3,5-tripropionate de tri(ⁿhexyle), le cyclohexane-1,3,5-tripropionate de tri(^{iso}hexyle), le cyclohexane-1,3,5-tripropionate de tri(ⁿheptyle), le cyclohexane-1,3,5-tripropionate de tri(^{iso}heptyle), le cyclohexane-1,3,5-tripropionate de tri(ⁿoctyle), le cyclohexane-1,3,5-tripropionate de tri(^{iso}octyle), le cyclohexane-1,3,5-tripropionate de tri(2-éthylhexyle), le cyclohexane-1,3,5-tripropionate de tri(ⁿnonyle), le cyclohexane-1,3,5-tripropionate de tri(^{iso}nonyle), le cyclohexane-1,3,5-tripropionate de tri(ⁿdécyle), le cyclohexane-1,3,5-tripropionate de tri(^{iso}décyle) et le cyclohexane-1,3,5-tripropionate de tri(2-propylheptyle) .

8. Plastifiant pour des polymères comprenant un triester de l'acide cyclohexanetripropionique selon l'une quelconque des revendications 1 à 4 et 7 ou un mélange selon l'une quelconque des revendications 5 à 7 et éventuellement au moins un autre composé plastifiant les polymères.

9. Composition, contenant un triester de l'acide cyclohexanetripropionique selon l'une quelconque des revendications 1 à 4 et 7 ou un mélange selon l'une quelconque des revendications 5 à 7 ou un plastifiant selon la revendication 8 et un ou plusieurs polymères.

10. Composition selon la revendication 9, **caractérisée en ce qu'**au moins un polymère est choisi dans le groupe constitué par le poly(chlorure de vinyle), le poly(méthacrylate d'alkyle) (PAMA), le polyvinylbutyral (PVB), le polyuréthane, le polysulfure, le poly(acide lactique) (PLA), le polyhydroxybutyral (PHB), la nitrocellulose et les copolymères du chlorure de vinyle avec de l'acétate de vinyle ou de l'acrylate de butyle.

11. Composition selon l'une quelconque des revendications 9 ou 10, **caractérisée en ce qu'**il s'agit d'un constituant d'un adhésif, d'une masse de bouchage, d'une masse de revêtement, d'une laque, d'une peinture, d'un plastisol, d'un mélange sec, d'une mousse, d'un cuir synthétique, d'un revêtement de sol, en particulier de sa couche de recouvrement ou de mousse, d'une bande de toit, d'une protection de soubassement, d'un revêtement de tissu, d'un câble, d'une isolation de fil, d'un tuyau, d'une particule extrudée, d'une feuille, d'un objet dans le domaine des intérieurs de voiture, d'un tapis, d'une encre, d'un jouet, d'une feuille de contact, d'un emballage alimentaire ou d'un article médical, en particulier d'un tuyau ou d'une poche à sang.

12. Utilisation d'un triester de l'acide cyclohexanetripropionique selon l'une quelconque des revendications 1 à 4 et 7 ou d'un mélange selon l'une quelconque des revendications 5 à 7 comme plastifiant pour des polymères, en particulier pour le PVC.

13. Utilisation selon la revendication 12, **caractérisée en ce qu'**un triester de l'acide cyclohexanetripropionique, dont les parties alcool comprennent à chaque fois 7 à 12 atomes de carbone, est utilisé comme composé plastifiant les polymères dans des applications haute température.

14. Utilisation selon la revendication 12, **caractérisée en ce qu'**un triester de l'acide cyclohexanetripropionique, dont les parties alcool comprennent à chaque fois 2 à 9 atomes de carbone, est utilisé comme composé plastifiant les polymères dans des applications de plastisol.

15. Préparation de triesters de l'acide cyclohexanetripropionique selon l'une quelconque des revendications 1 à 4 et 7 par
- hydrogénation du noyau des triesters correspondants de l'acide tripropionique,
- transestérification de l'ester trialkylique de l'acide cyclohexanetripropionique présentant au moins un alcool comprenant 2, 3, 4, 5, 6, 7, 8, 9, 10, 11 ou 12 atomes de carbone, l'alcool de la partie alcool à incorporer de l'acide trialkylique présentant un point d'ébullition supérieur à celui de l'alcool de la partie alcool qui doit être remplacée dans le cadre de la transestérification,
- estérification de l'acide cyclohexanetripropionique à l'aide d'au moins un alcool comprenant 2, 3, 4, 5, 6, 7, 8, 9, 10, 11 ou 12 atomes de carbone ou
- alcoxycarbonylation de trivinylcyclohexane à l'aide d'au moins un alcool comprenant 2, 3, 4, 5, 6, 7, 8, 9, 10, 11 ou 12 atomes de carbone.
